(19) Europäisches Patentamt
European Patent Office
Office européen des brevets

(11) Numéro de publication : **0 178 201**
**B1**

(12) # FASCICULE DE BREVET EUROPÉEN

(45) Date de publication du fascicule du brevet : **01.02.89**

(21) Numéro de dépôt : **85401732.4**

(22) Date de dépôt : **06.09.85**

(51) Int. Cl.[4] : **C 07 D491/04**, A 61 K 31/435// C07D211/72, C07D211/74 ,(C07D491/04, 307:00, 221:00)

(54) **Dérivés de furo [3,2-c]pyridines, leur préparation et leur application en thérapeutique.**

(30) Priorité : **27.09.84 FR 8414842**

(43) Date de publication de la demande : **16.04.86 Bulletin 86/16**

(45) Mention de la délivrance du brevet : **01.02.89 Bulletin 89/05**

(84) Etats contractants désignés : **AT BE CH DE FR GB IT LI LU NL SE**

(56) Documents cités :
**EP-A- 0 088 250**
**FR-A- 2 215 948**

(73) Titulaire : **SYNTHELABO**
**58, rue de la Glacière**
**F-75621 Paris Cedex 13 (FR)**

(72) Inventeur : **Wick, Alexander**
**10, Boulevard des Plants**
**F-78860 St.Nom La Breteche (FR)**
Inventeur : **Frost, Jonathan**
**7, Route de Montjean**
**F-91320 Wissous (FR)**
Inventeur : **Bertin, Jean**
**55, rue d'Estienne d'Orves**
**F-92140 Clamart (FR)**

(74) Mandataire : **Ludwig, Jacques et al**
**SYNTHELABO Service Brevets 58, rue de la Glacière**
**F-75621 Paris Cedex 13 (FR)**

**EP 0 178 201 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention a pour objet des dérivés de furo [3,2-c] pyridines, leur préparation et leur application en thérapeutique.

Les composés de l'invention répondent à la formule générale I

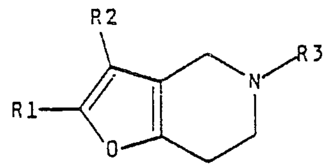

(I)

dans laquelle

R1 représente un groupe phényle, halophényle, méthylphényle, éthylphényle, méthoxyphényle, trifluorométhylphényle ou naphtyle,

R2 représente l'hydrogène ou un groupe méthyle ou phényle, et

R3 représente l'hydrogène ou un groupe benzyle,

étant exclus les composés dans la formule générale I desquels R1 représente un groupe phényle ou fluoro-4 phényle, R2 représente l'hydrogène et R3 représente l'hydrogène ou un groupe benzyle.

Ces composés exclus sont en effet décrits comme intermédiaires de synthèse dans un article de Chem. Pharm. Bull., 1977, 25 (8), 1911-1922, résumé dans CA 88 : 22555d.

Les sels d'addition que peuvent former les composés de formule générale I font également partie de l'invention.

Conformément à l'invention, on peut préparer les composés de formule générale I selon le schéma de la page suivante. On soumet une oxo-4 pipéridine de formule générale II, dans laquelle R4 représente un groupe benzyle, benzoyle ou acétyle, à l'action de la pyrrolidine, éventuellement en présence d'acide p-toluènesulfonique, dans un solvant tel que le toluène.

On obtient ainsi un composé de formule générale III, que l'on fait réagir avec une cétone α-bromée de formule générale IV, dans laquelle R1 et R2 sont tels que définis ci-dessus, à froid, et dans un solvant tel que le benzène ou l'acétonitrile.

Schéma

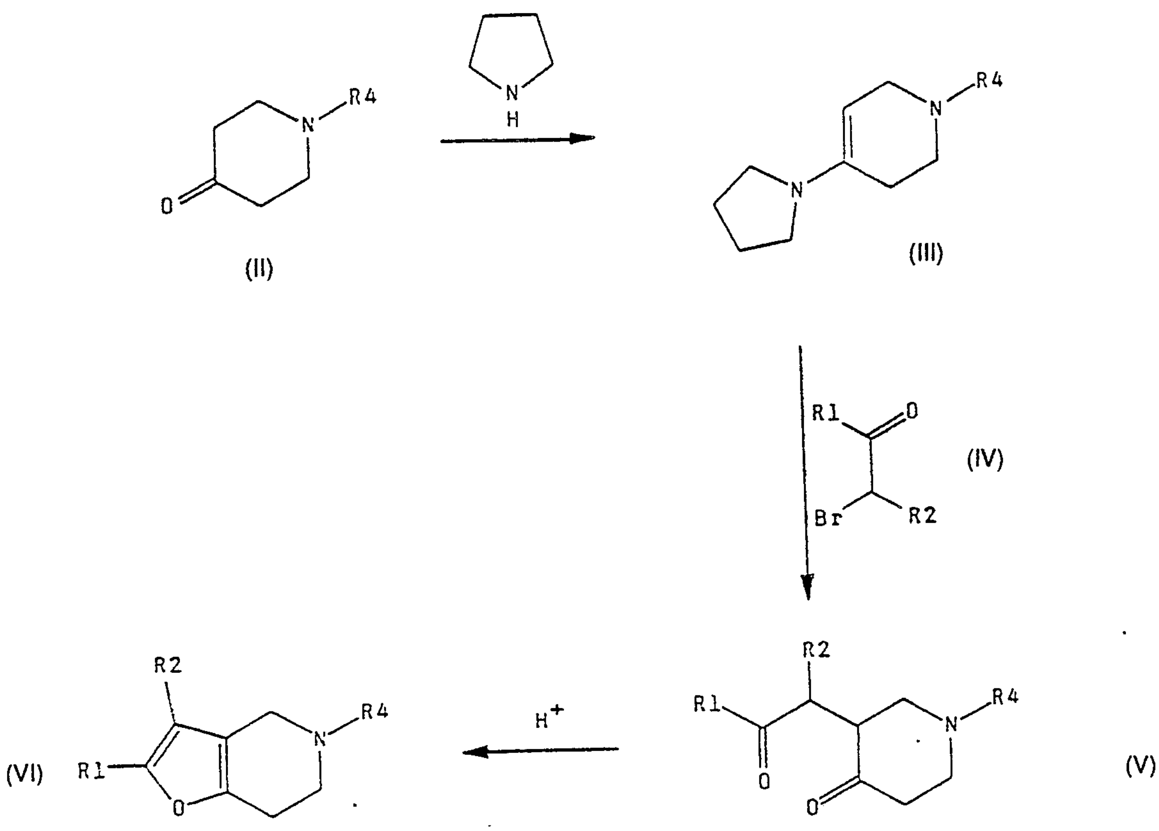

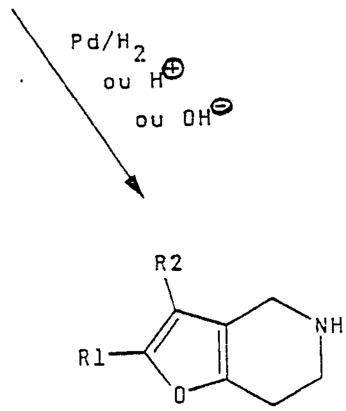


(VII)

Ensuite on effectue la cyclisation du composé de formule générale V ainsi obtenu en milieu acide chlorhydrique concentré ou en présence d'acide p-toluènesulfonique et à la température de reflux. On obtient une furopyridine de formule générale VI qui, lorsque R4 représente un groupe benzyle, répond également à la formule générale I.

Ce composé peut ensuite être déprotégé, soit par hydrogénation catalytique dans le cas où R4 représente un groupe benzyle, soit par action d'un acide ou d'une base forte lorsque R4 représente un groupe acétyle ou benzoyle. Dans le cas de l'acide, son action peut ne constituer qu'une seule étape avec l'étape précédente de cyclisation du composé V.

Les exemples suivants illustrent la préparation de quelques composés selon l'invention.

La microanalyse et les spectres IR et RMN confirment les structures des composés obtenus.

Exemple 1. (Naphtyl-2)-2 benzyl-5 tétrahydro-4,5,6,7 furo [3,2-c] pyridine

a) Benzyl-1 (pyrrolidinyl-1)-4 tétrahydro-1,2,3,6 pyridine.

Dans un ballon de 1 000 ml muni d'un appareil de Dean-Stark, d'un réfrigérant, d'une garde à chlorure de calcium, d'un agitateur magnétique et d'un chauffage à bain d'huile, on introduit 101 g (0,53 mole) de benzyl-1 pipéridinone-4, 200 ml de toluène, 56,9 g (0,8 mole) de pyrrolidine et 30 mg d'acide p-toluènesulfonique.

On chauffe le mélange au reflux pendant environ 5 heures, jusqu'à ce que l'eau formée soit complètement distillée. Puis on refroidit le mélange sur bain de glace, et on évapore le solvant. Il reste une huile orangée qu'on utilise telle quelle pour la suite.

b) Benzyl-1 [(naphtyl-2) carbonylméthyl]-3 pipéridinone-4.

Dans un erlenmeyer de 250 ml muni d'une ampoule isobare, d'une garde à chlorure de calcium et d'un agitateur magnétique, on introduit 13 g (0,0535 mole) de l'huile précédemment obtenue, 65 ml de benzène, et on refroidit la solution sur bain de glace.

Puis on ajoute lentement une solution de 12,45 g (0,05 mole) de bromo-2 (naphtyl-2)-1 éthanone dans 70 ml de benzène. Après 30 minutes d'agitation à 0 °C, on laisse revenir le mélange à la température ambiante et on le laisse reposer une nuit.

On verse le produit huileux dans un mélange de 200 ml d'eau et 400 ml d'acétate d'éthyle, on sépare une fraction insoluble par filtration, on sépare la phase organique du filtrat, on la lave à l'eau, on la sèche sur sulfate de magnésium et on la concentre. Il reste une huile brune qu'on utilise telle quelle pour la suite.

c) (Naphtyl-2)-2 benzyl-5 tétrahydro-4,5,6,7 furo [3,2-c] pyridine.

Dans un ballon de 1 000 ml muni d'un réfrigérant, d'un agitateur magnétique et d'un chauffage à bain d'huile on introduit 15,2 g (0,0425 mole) de l'huile précédemment obtenue et 150 ml d'acide chlorhydrique concentré (à 37 %). On chauffe le mélange au reflux pendant 2 heures, on le laisse refroidir, on sépare des cristaux bruns par filtration et on les lave à l'eau. On purifie la base par chromatographie sur silice en éluant avec un mélange 98/2 de chlorure de méthylène/acétate d'éthyle, et on recristallise dans l'éthanol. Point de fusion : 140-142 °C.

Exemple 2. Phényl-2 méthyl-3 benzyl-5 tétrahydro-4,5,6,7 furo [3,2-c] pyridine et son chlorhydrate

a) Benzyl-1 (benzoyl-1 éthyl)-3 pipéridinone-4.

Dans un appareil tel que décrit sous 1b) on introduit 13 g (0,0535 mole) de benzyle-1 (pyrrolidinyl-1)-4 tétrahydro-1,2,3,6 pyridine (préparée selon l'exemple 1a), 65 ml de benzène et on refroidit le mélange + 0 °C sur bain de glace.

On ajoute lentement une solution de 12,5 g (0,0528 mole) de bromo-2 phényl-1 propanone-1 dans

50 ml de benzène. Après 30 minutes d'agitation à 0 °C, on laisse revenir le mélange à température ambiante, on le chauffe lentement jusque 50 °C et, après 1 heure, on le laisse refroidir. On ajoute 100 ml d'eau, et 50 ml d'acétate d'éthyle, on agite, on sépare la phase organique, on la lave, on la sèche et on chasse le solvant. Il reste une huile brune qu'on utilise telle quelle pour la suite.

b) Phényl-2 méthyl-3 benzyl-5 tétrahydro-4,5,6,7 furo [3,2-c] pyridine.

Dans un ballon de 500 ml muni d'un réfrigérant, d'un agitateur magnétique et d'un chauffage à bain d'huile, on introduit 20,4 g de l'huile impure précédemment obtenue et 200 ml d'acide chlorhydrique concentré (37 %), et on chauffe au reflux pendant 1 h 30.

Après une nuit à température ambiante, on verse le mélange sur de l'eau glacée, on ajoute de l'ammoniaque jusqu'à pH > 7, on ajoute de l'acétate d'éthyle, et on agite jusqu'à dissolution complète du solide gommeux.

On sépare la phase organique, on la lave, on la sèche et on l'évapore.

Il reste une huile brune qu'on chromatographie sur colonne de silice avec du chlorure de méthylène.

On obtient finalement des cristaux orangés qui fondent à environ 65 °C.

On en prépare le chlorhydrate en les dissolvant dans de l'éther et en ajoutant, sur bain de glace, de l'éther chlorhydrique. On filtre les cristaux qui se forment, on les lave à l'éther, on les sèche et on les recristallise dans l'éthanol. Point de fusion : 235-237 °C.

Exemple 3. (Naphtyl-2)-2 tétrahydro-4,5,6,7 furo [3,2-c] pyridine et son maléate

a) Acétyl-1 (pyrrolidinyl-1)-4 tétrahydro-1,2,3,6 pyridine.

Dans un appareil tel que décrit sous 1a) on introduit 98,7 g (0,7 mole) d'acétyl-1 pipéridinone-4, 200 ml de toluène, 75,2 g (1,06 mole) de pyrrolidine et 30 mg d'acide p-toluènesulfonique.

On chauffe le mélange au reflux pendant 2 h 30 en séparant l'eau formée.

Après une nuit à température ambiante on chasse le solvant et on recueille des cristaux bruns huileux qu'on utilise tels quels pour la suite.

b) Acétyl-1 [(naphtyl-2) carbonylméthyl]-3 pipéridinone-4.

Dans un appareil tel que décrit sous 1b) on introduit 10,4 g (0,0535 mole) des cristaux bruns précédemment obtenus et 65 ml de benzène, et on refroidit le mélange sur bain de glace.

Puis on ajoute lentement une solution de 12,4 g (0,05 mole) de bromo-2 (naphtyl-2)-1 éthanone dans 70 ml de benzène. Après 30 minutes d'agitation à 0 °C, on laisse revenir le mélange à la température ambiante.

On le verse dans un mélange de 200 ml d'eau et 400 ml d'acétate d'éthyle, on sépare la phase organique, on la lave, on la sèche et on chasse le solvant. Il reste un produit orange qu'on utilise tel quel pour la suite.

c) (Naphtyl-2)-2 acétyl-5 tétrahydro-4,5,6,7 furo [3,2-c] pyridine.

Dans un appareil tel que décrit sous 1c) on introduit 11,4 g (0,037 mole) du produit précédemment obtenu, puis 115 ml d'acide chlorhydrique à 37 %.

On chauffe le mélange au reflux pendant 2 heures, on le laisse revenir à température ambiante et, après une nuit de repos, on le place sur bain de glace, on ajoute de l'ammoniaque à 20 % jusqu'à pH > 7, puis environ 500 ml d'acétate d'éthyle.

Après dissolution du produit on sépare la phase organique, on la lave, on la sèche et on évapore le solvant.

Il reste un mélange d'huile et de cristaux bruns que l'on purifie par chromatographie sur colonne de silice en éluant avec un mélange 98/2 de chlorure de méthylène/méthanol. On isole finalement des cristaux bruns qu'on utilise tels quels pour la suite.

d) (Naphtyl-2)-2 tétrahydro-4,5,6,7 furo [3,2-c] pyridine et son maléate.

Dans l'appareil utilisé précédemment on introduit 2,9 g (0,01 mole) des cristaux précédemment obtenus, 150 ml d'éthanol et 300 ml de soude 5N.

On chauffe au reflux pendant 2 h 30, et on laisse revenir à température ambiante.

On verse le mélange sur de l'eau glacée, on l'extrait avec de l'acétate d'éthyle, on filtre la phase organique, on la lave, on la sèche et on chasse le solvant. Il reste des cristaux bruns dont on prépare le maléate. Pour cela on en dissout 1,92 g dans 50 ml d'acétate d'éthyle et on leur ajoute lentement une solution de 0,98 g d'acide maléique dans 40 ml d'acétate d'éthyle. Il se forme des cristaux que l'on isole par filtration et qu'on recristallise dans 80 ml d'éthanol. On obtient des cristaux blancs.

Point de fusion : 190-192 °C (décomposition).

Exemple 4. (Méthyl-4 phényl)-2 tétrahydro-4,5,6,7 furo [3,2-c] pyridine et son chlorhydrate

a) Acétyl-1 (méthyl-4 benzoylméthyl)-3 pipéridinone-4.

Dans un ballon de 500 ml muni d'une ampoule isobare, d'une garde à chlorure de calcium et d'un agitateur magnétique, on introduit 20 g (0,103 mole) d'acétyl-1 (pyrrolidinyl-1)-4 tétrahydro-1,2,3,6 pyridine (préparée selon l'exemple 3a), 100 ml de benzène, et on ajoute lentement 20 g (0,0937 mole) de bromo-2 (méthyl-4 phényl)-1 éthanone en solution dans 100 ml de benzène.

On agite le mélange une nuit à température ambiante, on le reprend avec 100 ml d'eau et 150 ml

d'acétate d'éthyle, on sépare la phase organique, on la lave, on la sèche et on l'évapore. Il reste une huile brune qu'on chromatographie sur silice en éluant avec un mélange 95/5 de chlorure de méthyle/acétate d'éthyle.

L'un des deux produits isolés est la bis-(méthyl-4 benzoylméthyl)-3,5 acétyl-1 pipéridinone-4. L'autre est bien le composé recherché ; il se présente sous forme d'une huile orangée qu'on utilise telle quelle pour la suite.

b) (Méthyl-4 phényl)-2 tétrahydro-4,5,6,7 furo [3,2-c] pyridine et son chlorhydrate.

Dans un appareil tel que décrit sous 1c) on introduit 8 g (0,0293 mole) de l'huile précédemment obtenue et 60 ml d'acide chlorhydrique à 37 %.

On chauffe le mélange au reflux pendant 4 heures, on le refroidit sur bain de glace, on le filtre, on le lave avec de l'eau puis de l'éthanol et on le sèche.

Il reste des cristaux de couleur ocre que l'on recristallise dans l'éthanol après traitement au noir animal. Point de fusion : 289-290 °C (décomposition).

Exemple 5. (Méthyl-4 phényl)-2 méthyl-3 benzyl-5 tétrahydro-4,5,6,7 furo [3,2-c] pyridine et son chlorhydrate

a) Benzyl-1 [(méthyl-4 benzoyl)-1 éthyl]-3 pipéridinone-4.

Dans un ballon de 1 000 ml muni d'une ampoule isobare, d'une garde à chlorure de calcium et d'un agitateur magnétique on introduit 53,3 g (0,22 mole) de benzyl-1 (pyrrolidinyl-1)-4 tétrahydro-1,2,3,6 pyridine (préparée selon l'exemple 1a), 200 ml de benzène, et on ajoute lentement 45,4 g (0,2 mole) de bromo-2 (méthyl-4 phényl)-1 propanone-1 en solution dans 100 ml de benzène.

Après 24 heures d'agitation on chasse le solvant, on reprend le résidu avec 200 ml d'eau et 200 ml d'acétate d'éthyle, on sépare la phase organique, on la lave, on la sèche et on l'évapore. Il reste une huile brune qu'on utilise telle quelle pour la suite.

b) (Méthyl-4 phényl)-2 méthyl-3 benzyl-5 tétrahydro-4,5,6,7 furo [3,2-c] pyridine et son chlorhydrate.

Dans un ballon de 1 000 ml muni d'un réfrigérant, d'un agitateur magnétique et d'un chauffage à bain d'huile on introduit 81 g (0,2 mole) de l'huile précédemment obtenue et 500 ml d'acide chlorhydrique à 37 %.

On chauffe au reflux pendant 2 heures, on refroidit le mélange, on sépare la phase aqueuse et on reprend le précipité gommeux avec de l'eau, de l'ammonique et de l'acétate d'éthyle. On agite jusqu'à dissolution, on sépare la phase organique, on la lave, on la sèche et on l'évapore.

Il reste des cristaux huileux qu'on purifie en les éluant sur colonne de silice avec du chlorure de méthylène. On obtient des cristaux jaunes dont on prépare le chlorhydrate comme indiqué précédemment, et on recristallise ce dernier dans l'alcool isopropylique. Point de fusion : 232-234 °C.

Exemple 6. (Méthyl-4 phényl)-2 méthyl-3 tétrahydro-4,5,6,7 furo [3,2-c] pyridine et son chlorhydrate

Dans un flacon de Parr de 500 ml on place 3 g (0,009 mole) de (méthyl-4 phényl)-2 méthyl-3 benzyl-5 tétrahydro-4,5,6,7 furo [3,2-c] pyridine (base libre, préparée selon l'exemple 5b), 40 ml d'acide acétique, 40 ml d'eau et 0,3 g de charbon palladié à 5 %.

On chauffe à 50 °C sous une pression d'hydrogène d'environ 0,35 MPa pendant 1 h 30.

On filtre le catalyseur, on chasse le solvant, on reprend le résidu avec 100 ml d'eau, on ajoute de l'ammoniaque jusqu'à $pH > 7$, puis 100 ml d'acétate d'éthyle.

On sépare la phase organique, on la lave, on la sèche et on l'évapore.

Il reste des cristaux blancs dont on prépare le chlorhydrate comme indiqué précédemment. Point de fusion : 252-254 °C.

Exemple 7. (Chloro-4 phényl)-2 tétrahydro-4,5,6,7 furo [3,2-c] pyridine et son chlorhydrate

a) Benzoyl-1 (pyrrolidinyl-1)-4 tétrahydro-1,2,3,6 pyridine.

Dans un ballon de 1 000 ml muni d'un appareil de Dean-Stark, d'un réfrigérant, d'une garde à chlorure de calcium, d'un agitateur magnétique et d'un chauffage à bain d'huile, on introduit 94,7 g (0,466 mole) de benzoyl-1 pipéridinone-4, 200 ml de toluène et 52,5 g (0,74 mole) de pyrrolidine.

On chauffe au reflux jusqu'à la fin de l'élimination de l'eau (environ 3 heures). On laisse refroidir et on évapore le mélange. Il reste une huile orange.

b) Benzoyl-1 [(chloro-4 phényl) carbonylméthyl]-3 pipéridinone-4.

Dans un ballon de 2 l muni d'une ampoule isobare, d'une garde à chlorure de calcium et d'un agitateur magnétique, on introduit 64,1 g (0,25 mole) de l'huile précédente et 250 ml de benzène, et on refroidit le mélange par un bain de glace. On introduit très lentement une solution refroidie de 55,5 g (0,238 mole) de (chloro-4 phényle)-1 bromo-2 éthanone-1 dans 200 ml de benzène, on agite 2 heures à 0 °C et 1 heure à température ambiante.

On évapore le mélange, on reprend le résidu avec 200 ml d'eau et 400 ml d'acétate d'éthyle, on sépare la phase organique, on la sèche, on l'évapore, et on chromatographie le résidu sur silice avec un mélange 95/5 de dichlorométhane/acétate d'éthyle. On recueille finalement une huile orange.

c) (Chloro-4 phényl)-2 benzoyl-5 tétrahydro-4,5,6,7 furo [3,2-c] pyridine.

Dans un ballon de 500 ml muni d'un appareil de Dean-Stark, d'un réfrigérant, d'une garde à chlorure de calcium, d'un agitateur magnétique et d'un chauffage à bain d'huile, on introduit 10,5 g (0,029 mole) de l'huile précédente, 150 ml de toluène et 5 g d'acide p-toluènesulfonique.

On chauffe au reflux en éliminant l'eau qui se forme, pendant 3 heures. On refroidit le mélange et on l'évapore.

Il reste une huile noire qu'on reprend avec 100 ml d'eau, on rend le mélange basique avec de l'ammoniaque diluée, et on l'extrait avec 150 ml d'acétate d'éthyle. On sépare la phase organique, on la lave, on la sèche et on l'évapore.

On purifie le résidu brun par chromatographie sur silice en éluant avec un mélange 95/5 de dichlorométhane/acétate d'éthyle.

On recueille ainsi des cristaux jaunes qu'on recristallise dans l'alcool isopropylique. Il reste des cristaux blancs. Point de fusion : 156-158 °C.

d) (Chloro-4 phényl)-2 tétrahydro-4,5,6,7 furo [3,2-c] pyridine et son chlorhydrate.

Dans un ballon de 1 000 ml muni d'un réfrigérant, d'un agitateur magnétique et d'un chauffage à bain d'huile, on introduit 3,4 g (0,01 mole) des cristaux précédents, 150 ml d'éthanol et 300 ml de soude 5N. On chauffe le mélange au reflux pendant 1 heure, et on le laisse reposer une nuit à température ambiante. Il se forme deux phases.

On ajoute 1 l d'eau, ce qui provoque une cristallisation. On sépare les cristaux par filtration, on les reprend avec 200 ml d'acétate d'éthyle et 100 ml d'eau, on sépare la phase organique, on la sèche et on l'évapore. Il reste des cristaux de couleur ocre. On en dissout 2,3 g dans 50 ml d'acétate d'éthyle, on refroidit la solution à 0 °C et on ajoute lentement 15 ml d'éther chlorhydrique. Après 30 minutes à 0 °C on filtre les cristaux, on les lave à l'acétate d'éthyle, on les sèche et on les recristallise dans 120 ml d'éthanol. Point de fusion : 299-300 °C.

Exemple 8 (Bromo-4 phényl)-2 tétrahydro-4,5,6,7 furo [3,2-c] pyridine et son chlorhydrate

a) Acétyl-1 (pyrrolidinyl-1)-4 tétrahydro-1,2,3,6 pyridine.

Dans un ballon de 1 000 ml muni d'un appareil de Dean-Stark, d'un réfrigérant, d'une garde à chlorure de calcium, d'un agitateur magnétique et d'un chauffage à bain d'huile, on introduit 85,5 g (0,606 mole) d'acétyl-1 pipéridinone-4, 200 ml de toluène et 64,65 g (1,10 mole) de pyrrolidine.

On chauffe au reflux pendant 2 heures, en éliminant l'eau formée, on laisse reposer une nuit à température ambiante et on chasse le solvant. Il reste une huile orange.

b) Acétyl-1 [(bromo-4 phényl) carbonyl-méthyl]-3 pipéridinone-4.

Dans un ballon de 1 000 ml muni d'une ampoule isobare, d'une garde à chlorure de calcium et d'un agitateur magnétique, on introduit 38,85 g (0,2 mole) de l'huile orange précédente et 100 ml d'acétonitrile sec.

Après 1 heure d'agitation à 0 °C, on laisse revenir à température ambiante pendant une nuit.

On sépare une fraction insoluble par filtration, on concentre le filtrat, on le reprend avec 200 ml d'acétate d'éthyle et 200 ml d'eau, on sépare la phase organique, on la lave à l'eau, on la sèche et on l'évapore.

Il reste une huile brune qu'on chromatographie sur silice en éluant avec un mélange 95/5 de dichlorométhane/acétate d'éthyle.

On recueille finalement une huile orange.

c) (Bromo-4 phényl)-2 acétyl-5 tétrahydro-4,5,6,7 furo [3,2-c] pyridine.

Dans un ballon de 500 ml muni d'un appareil de Dean-Stark, d'un réfrigérant, d'une garde à chlorure de calcium, d'un agitateur magnétique et d'un chauffage à bain d'huile, on introduit 24,8 g (0,073 mole) de l'huile précédente, 200 ml de toluène et 13,95 g d'acide p-toluènesulfonique, et on chauffe au reflux pendant 2 h 30 en éliminant l'eau.

On laisse le mélange revenir à température ambiante puis on le refroidit par un bain de glace. Il se forme des cristaux qu'on sépare par filtration.

On les reprend avec 100 ml d'eau et 800 ml d'acétate d'éthyle, on sépare une fraction insoluble, on sépare la phase organique, on la lave à l'eau, on la sèche et on l'évapore.

Après recristallisation dans l'éthanol, on obtient des cristaux blancs. Point de fusion : 194-196 °C.

d) (Bromo-4 phényl)-2 tétrahydro-4,5,6,7 furo [3,2-c] pyridine et son chlorhydrate.

Dans un ballon de 1 000 ml muni d'un réfrigérant, d'un agitateur magnétique et d'un chauffage à bain d'huile, on introduit 11,26 g (0,035 mole) des cristaux précédents, 150 ml d'éthanol et 300 ml de soude 5N.

On chauffe lentement au reflux, on laisse refroidir et après 1 h 30 on refroidit le mélange par un bain de glace.

On filtre les cristaux formés, on les lave à l'eau, on les reprend avec 60 ml d'acétate d'éthyle, on lave la solution à l'eau, on la sèche, on l'évapore, et on chromatographie le résidu avec un mélange 95/5 de dichlorométhane/méthanol. On dissout 3 g de la base pure ainsi obtenue dans 50 ml d'acétate d'éthyle, et on ajoute 25 ml d'éther chlorhydrique. On essore les cristaux formés, on les lave à l'acétate d'éthyle, et on les recristallise dans l'éthanol. Point de fusion 270-272 °C.

Le tableau ci-après illustre les structures et points de fusion de divers composés selon l'invention.

Tableau

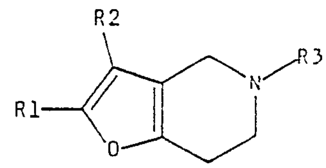

(I)

| Composé | Exemple | R1 | R2 | R3 | Sel/ base | F(°C) |
|---|---|---|---|---|---|---|
| 1 | 1 | Naphtyle-2 | H | $CH_2C_6H_5$ | 00 | 140-142 |
| 2 | | $4-CH_3O-C_6H_4$ | H | $CH_2C_6H_5$ | 10 | 255-257 |
| 3 | 2 | $C_6H_5$ | $CH_3$ | $CH_2C_6H_5$ | 10 | 235-237 |
| 4 | | $4-Br-C_6H_4$ | H | $CH_2C_6H_5$ | 10 | 271-272 |
| 5 | 3 | Naphtyle-2 | H | H | 14 | 190-192(d) |
| 6 | | $4-CH_3-C_6H_4$ | H | $CH_2C_6H_5$ | 10 | 278-280 |
| 7 | | $2-CH_3-C_6H_4$ | H | $CH_2C_6H_5$ | 10 | 258-259 |
| 8 | 4 | $4-CH_3-C_6H_4$ | H | H | 10 | 289-290(d) |
| 9 | | $2-CH_3-C_6H_4$ | H | H | 10 | 244-246 |
| 10 | | $4-Cl-C_6H_4$ | H | $CH_2C_6H_5$ | 10 | 265-267 |
| 11 | | $3-CH_3-C_6H_4$ | H | H | 10 | 272-275 |
| 12 | 5 | $4-CH_3-C_6H_4$ | $CH_3$ | $CH_2C_6H_5$ | 10 | 232-234 |
| 13 | 6 | $4-CH_3-C_6H_4$ | $CH_3$ | H | 10 | 252-254 |
| 14 | | $2-F-C_6H_4$ | H | H | 10 | 282-284 |
| 15 | | $3-CF_3-C_6H_4$ | H | H | 10 | 270-272 |
| 16 | | $4-C_2H_5-C_6H_4$ | H | H | 10 | 282-282 |
| 17 | | $3-C_2H_5-C_6H_4$ | H | H | 10 | 264-266 |
| 18 | 7 | $4-Cl-C_6H_4$ | H | H | 10 | 299-300 |
| 19 | | $2-Cl-C_6H_4$ | H | H | 10 | 265-267 |
| 20 | | $4-CH_3O-C_6H_4$ | H | H | 10 | 268-269 |
| 21 | 8 | $4-Br-C_6H_4$ | H | H | 10 | 270-272 |

Sel ou base : 00 base
10 chlorhydrate
14 maléate
(d) : fusion avec décomposition

Les composés de l'invention ont fait l'objet d'une série d'essais pharmacologiques qui ont mis en évidence leur intérêt comme substances thérapeutiques.

Toxicité

La toxicité aiguë a été déterminée chez des souris de souche CD1 par une méthode graphique.

Pour la plupart des composés la DL 50 (dose léthale 50 %) est supérieure à 1 000 mg/kg par voie intrapéritonéale, et par voie orale elle est de 100 à plus de 1 000 mg/kg.

Activité antidépressive

Les composés de l'invention ont fait l'objet d'un essai de liaison (binding) de $^3$H-tryptamine sur le cortex total de rat.

On utilise des rats mâles Sprague-Dawley de 150 à 200 g. Après dislocation cervicale on excise le cerveau et on dissèque le cortex cérébral sur une boîte de culture refroidie par de la glace.

On homogénéise le tissu dans 50 ml de mélange tampon (50 mM de Tris, pH = 7,0, à 25 °C, titrant 5 mM d'acide ascorbique et 230 μM de pargyline), à l'aide d'un mélangeur Polytron® (15 secondes à vitesse 6), et on le centrifuge pendant 10 mn à 48 000 × g et à 4 °C.

On dilue le culot dans 50 volumes de tampon, on le centrifuge à nouveau, et on met le culot final en suspension dans 19 ml de tampon.

On fait incuber des portions aliquotes (150 μl ; 0,3 mg de protéine) de suspension de membranes, pendant 60 mn à 0 °C avec de la $^3$H-tryptamine (activité spécifique) 39,5 Ci/millimole) dans un volume final de 1 ml, avec un tampon fraîchement préparé.

On achève l'incubation par filtration rapide à travers des filtres en fibre de verre Whatman GF/B, on lave les filtres trois fois avec 4 ml de tampon, on les sèche et on les mesure par spectroscopie de scintillation liquide.

La liaison spécifique correspond à la différence de radio-activité des membranes en l'absence et en présence de 10 μM de tryptamine non marquée dans le mélange d'incubation. Pour les déplacements par les composés à étudier, la concentration de $^3$H-tryptamine est de 3,3 nM et, à l'aide de différentes concentrations de composés à étudier, on détermine graphiquement la concentration $IC_{50}$, concentration du composé étudié qui inhibe 50 % de la liaison spécifique de $^3$H-tryptamine.

Les concentrations $IC_{50}$ des composés de l'invention sont pour la plupart de l'ordre de 0,5 μM, et de moins de 0,05 μM pour certains d'entre eux.

Les composés de l'invention ont aussi fait l'objet d'un essai de liaison (binding) de $^3$H-imipramine sur le cortex total de rat.

La préparation du tissu comprend :

a) Une homogénéisation dans 50 volumes (par gramme de tissu) de tampon d'incubation, suivie d'une centrifugation à 20 000 t/mn pendant 10 mn.

b) Une répétition de l'opération précédente avec le culot de centrifugation.

c) Une reprise de culot final dans 33 volumes (par gramme de tissu) dans le tampon d'incubation, d'où une suspension de 30 mg de tissu par ml.

Le tampon d'incubation contient, par litre, 120 millimoles de chlorure de sodium, 50 millimoles de Tris et 5 millimoles de chlorure de potassium, et présente un pH de 7,4 à 0 °C. Pour la détermination de la liaison totale, on introduit dans chaque tube d'incubation

100 μl de membrane de cortex à 30 mg/ml,
150 μl de tampon, et
50 μl de $^3$H-imipramine.

Pour la détermination de la liaison non spécifique, on introduit dans chaque tube d'incubation

100 μl de membrane de cortex à 30 mg/ml,
140 μl de tampon,
10 μl de désipramine à $30 \cdot 10^{-4}$ moles/l, et
50 μl de $^3$H-imipramine.

Pour les déplacements par les substances à étudier, la concentration de $^3$H-imipramine est de 2,5 nM, et celles des substances actives vont jusqu'à 100 μM.

L'incubation est effectuée à 0 °C pendant 1 heure.

Pour évaluer l'activité des composés on établit une courbe du pourcentage d'inhibition de la liaison spécifique de la $^3$H-imipramine en fonction de la concentration en drogue déplaçante. L'$IC_{50}$ est la concentration en drogue déplaçante qui inhibe 50 % de la liaison spécifique $^3$H-imipramine (détermination graphique) et à la concentration de ligand tritié de 2,5 nM.

Les concentrations $IC_{50}$ des composés de l'invention sont pour la plupart de l'ordre de 1 μM, et descendent jusqu'à 0,012 μM pour certains d'entre eux.

Les composés de l'invention ont également fait l'objet d'un essai quant à leur inhibition de la capture de la noradrénaline et de la sérotonine dans une préparation de synaptosomes non purifiés d'hypothalamus de rats, essai inspiré de la méthode de Ross S.B. et Renyi A.L. (Acta Pharmacolog. Toxicol., 36, 382-394, 1975).

Chaque mélange d'incubation comprend 0,1 nmole de $^{3}H$-noradrénaline, 0,1 nmole de $^{14}C$-sérotonine, 100 μl de la préparation de synaptosomes non purifiés (correspondant à 10 mg de tissu cérébral initial), le composé à étudier, et 1,8 ml de tampon de Krebs-Henseleit (pH 7,4, 1,25 μmole de nialamide, 114 μmoles d'acide ascorbique, 59,5 μmoles d'EDTA disodique et 1 111 μmoles de glucose pour 100 ml de tampon). Le processus de capture est déclenché par l'addition des amines marquées, et l'incubation est effectuée pendant 5 mn dans des tubes de centrifugation maintenus à 37 °C. On interrompt la réaction en filtrant le milieu d'incubation sur des filtres millipore MF (mélange d'esters de cellulose) de 0,45 μm (ref. HAWP 02500) puis en rinçant avec 2,5 ml de sérum physiologique. On introduit ensuite les filtres secs qui ont retenu les synaptosomes dans des flacons à scintillation, on ajoute 10 ml de liquide scintillant (Aquasol-NEN). On laisse reposer pendant 2 heures au cours desquelles les filtres sont dissouts puis on mesure la radio-activité.

La capture effective est obtenue en retranchant la capture passive dans les mêmes conditions mais dans la glace. L'inhibition de la capture est déterminée en pourcentage de la capture effective, avec et sans le composé à étudier, dans le même essai. La concentration $IC_{50}$, c'est-à-dire la concentration (en μM) qui provoque 50 % d'inhibition, est établie à partir d'une courbe semilogarithmique de la réponse en fonction de la concentration.

La concentration $IC_{50}$ des composés de l'invention va de 0,15 à 1,68 μM dans le cas de la noradrénaline et de 0,058 à 12 μM dans le cas de la sérotonine.

Les composés ont également été soumis à un essai de potentialisation des « head-twitches » (ébrouements de la tête) provoqués par le L-5-hydroxy-tryptophane chez la souris. Les souris (mâles CD1, Charles River France ; 18-22 g de poids corporel) reçoivent les produits à étudier, à doses croissantes, ou le solvant, simultanément avec le L-5-HTP à la dose de 125 mg/kg, par voie sous-cutanée. 30 minutes après cette injection de L-5-HTP, le nombre d'ébrouements de la tête est compté, pour chaque souris, pendant une minute. Pour chaque traitement, la moyenne des « head-twitches », ainsi que le pourcentage de variation par rapport au lot témoin, sont calculés.

A partir de la courbe effet-dose, on détermine la DA 100 (dose active 100 % ou dose qui augmente de 100 % le nombre moyen d'ébrouements), par la méthode graphique de Miller et Tainter (1944).

Les DA 100 des composés de l'invention varient de 1 à 30 mg/kg par voie intrapéritonéale.

Activité anti-ischémique

Les composés de l'invention ont été soumis au test de l'ischémie cérébrale complète.

L'ischémie est due à un arrêt cardiaque induit par une injection intraveineuse rapide de chlorure de magnésium. Dans ce test on mesure le « temps de survie », c'est-à-dire l'intervalle entre le moment de l'injection de chlorure de magnésium et le dernier mouvement respiratoire observable de chaque souris. Ce dernier mouvement est considéré comme l'indice ultime d'une fonction du système nerveux central. L'arrêt respiratoire apparaît approximativement 19 secondes après l'injection de chlorure de magnésium.

Des souris mâles (Charles River CD1) sont étudiées par groupes de 10.

Les souris sont nourries et abreuvées ad libitum avant les essais. Le temps de survie est mesuré 10 minutes après l'administration intrapéritonéale des composés de l'invention. Les résultats sont donnés sous la forme de la différence entre le temps de survie mesuré dans un groupe de 10 souris ayant reçu le composé et le temps de survie mesuré dans un groupe de 10 souris ayant reçu le liquide véhicule. Les rapports entre les modifications dans le terme de survie et la dose du composé sont enregistrés graphiquement selon une courbe semilogarithmique.

Cette courbe permet le calcul de la dose effective 3 secondes ($DE_{3''}$), c'est-à-dire la dose (en mg/kg) qui produit une augmentation de 3 secondes du temps de survie par rapport au groupe témoin de 10 souris non traitées.

Une augmentation de 3 secondes dans le temps de survie est à la fois significative statistiquement et reproductible. Les $DE_{3''}$ des composés de l'invention vont de 10 à 60 mg/kg par voie intrapéritonéale.

Enfin les composés de l'invention ont été soumis au test de l'hypoxie hypobare.

Des souris de souche CD1 sont maintenues dans une atmosphère appauvrie en oxygène, par réalisation d'un vide partiel (190 mm de mercure correspondant à 5,25 % d'oxygène).

Le temps de survie des animaux est noté. Ce temps est augmenté par les agents capables de favoriser l'oxygénation tissulaire et en particulier cérébrale. Les composés étudiés sont administrés, à plusieurs doses, par voie intrapéritonéale, 10 minutes avant l'essai. Les pourcentages d'augmentation du temps de survie par rapport aux valeurs obtenues chez les animaux témoins sont calculés. La dose active moyenne (DAM), dose qui augmente le temps de survie de 100 %, est déterminée graphiquement.

Les DAM de quelques composés de l'invention se situent entre 10 et 30 mg/kg par voie intrapéritonéale.

L'étude pharmacologique des composés de l'invention montre qu'ils possèdent une activité antidépressive et anti-ischémique. Ils peuvent être utilisés en thérapeutique pour le traitement des troubles de la vigilance, en particulier pour lutter contre les troubles du comportement imputables à des dommages vasculaires cérébraux et à la sclérose cérébrale en gériatrie, ainsi que pour le traitement des encéphalopathies métaboliques, et pour le traitement des états dépressifs.

L'invention comprend, par conséquent, toutes compositions pharmaceutiques renfermant les

composés et/ou leurs sels comme principes actifs, en association avec tous excipients appropriés à leur administration, en particulier par voie orale ou parentérale.

Les voies d'administration peuvent être les voies orale et parentérale.

La posologie quotidienne peut aller de 1 à 100 mg par voie parentérale et de 5 à 500 mg par voie orale.

**Revendications** (pour les Etats contractants : BE, CH, DE, FR, GB, IT, LI, LU, NL, SE)

1. Composés répondant à la formule générale I

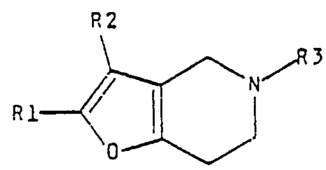

(I)

dans laquelle

R1 représente un groupe phényle, halophényle, méthylphényle, éthylphényle, méthoxyphényle, trifluorométhylphényle ou naphtyle,

R2 représente l'hydrogène ou un groupe méthyle ou phényle, et

R3 représente l'hydrogène ou un groupe benzyle,

à l'exception des composés dans la formule desquels R1 représente un groupe phényle ou fluoro-4 phényle, R2 représente l'hydrogène et R3 représente l'hydrogène ou un groupe benzyle, ainsi que leurs sels d'addition à des acides pharmaceutiquement acceptables.

2. Procédé de préparation des composés selon la revendication 1, caractérisé en ce qu'on soumet une oxo-4 pipéridine de formule générale II

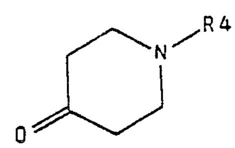

(II)

dans laquelle R4 représente un groupe benzyle, benzoyle ou acétyle, à l'action de la pyrrolidine, obtenant ainsi un composé de formule générale III

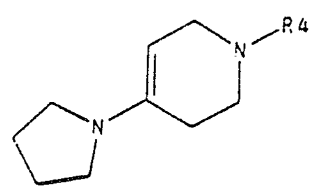

(III)

que l'on fait réagir avec une cétone α-bromée de formule générale IV

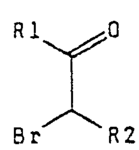

(IV)

dans laquelle R1 et R2 sont tels que définis dans la revendication 1, puis on effectue la cyclisation du composé de formule générale V ainsi obtenu

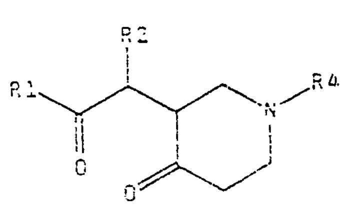

(V)

de manière à obtenir une furopyridine de formule générale VI

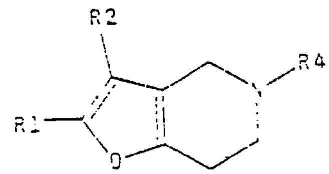

(VI)

et, lorsque R4 représente un groupe acétyle ou benzoyle on soumet le composé VI à l'action d'un acide ou d'une base forte pour éliminer le groupe acétyle ou bien, lorsque R4 représente un groupe benzyle, et si on le désire, on soumet le composé VI à une hydrogénation catalytique.

3. Composition pharmaceutique caractérisée en ce qu'elle contient un composé selon la revendication 1 en association avec un excipient pharmaceutique approprié.

**Revendication** (pour l'Etat contractant AT)

Procédé de préparation de composés répondant à la formule générale I

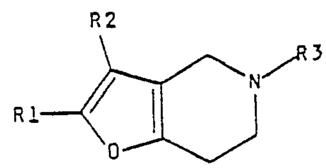

(I)

dans laquelle

R1 représente un groupe phényle, halophényle, méthylphényle, éthylphényle, méthoxyphényle, trifluorométhylphényle ou naphtyle,

R2 représente l'hydrogène ou un groupe méthyle ou phényle, et

R3 représente l'hydrogène ou un groupe benzyle,

à l'exception des composés dans la formule desquels R1 représente un groupe phényle ou fluoro-4 phényle, R2 représente l'hydrogène et R3 représente l'hydrogène ou un groupe benzyle, procédé caractérisé en ce qu'on soumet une oxo-4 pipéridine de formule générale II

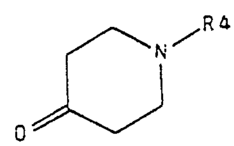

(II)

dans laquelle R4 représente un groupe benzyle, benzoyle ou acétyle, à l'action de la pyrrolidine, obtenant ainsi un composé de formule générale III

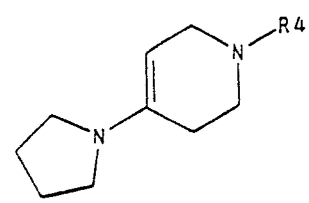

(III)

que l'on fait réagir avec une cétone α-bromée de formule générale IV

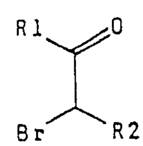

(IV)

dans laquelle R1 et R2 sont tels que définis ci-dessus, puis on effectue la cyclisation du composé de formule générale V ainsi obtenu

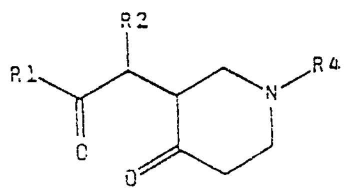

(V)

de manière à obtenir une furopyridine de formule générale VI

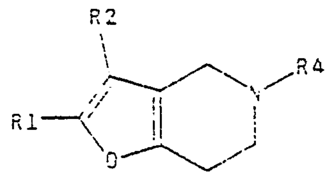

(VI)

et, lorsque R4 représente un groupe acétyle ou benzoyle on soumet le composé VI à l'action d'un acide ou d'une base forte pour éliminer le groupe acétyle ou bien, lorsque R4 représente un groupe benzyle, et si on le désire, on soumet le composé VI à une hydrogénation catalytique.

**Claims** (for the Contracting States : BE, CH, DE, FR, GB, IT, LI, LU, NL, SE)

1. Compounds corresponding to the general formula I

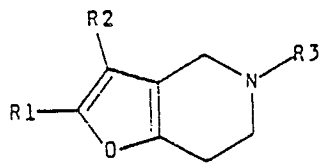

(I)

in which
R1 denotes a phenyl, halophenyl, methylphenyl, ethylphenyl, methoxyphenyl, trifluoromethylphenyl or naphthyl group,
R2 denotes hydrogen or a methyl or phenyl group, and
R3 denotes hydrogen or a benzyl group,
with the exception of the compounds in the formula of which R1 denotes a phenyl or 4-fluorophenyl group, R2 denotes hydrogen and R3 denotes hydrogen or a benzyl group, as well as their addition salts with pharmaceutically acceptable acids.

2. Process for preparing the compounds according to Claim 1, characterised in that a 4-oxopiperidine of the general formula II

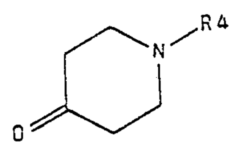

(II)

in which R4 denotes a benzyl, benzoyl or acetyl group, is subjected to the action of pyrrolidine, a compound of the general formula III

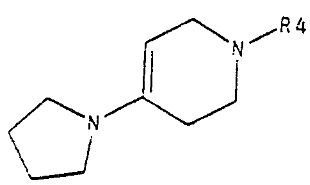

(III)

thereby being obtained, which is reacted with an α-brominated ketone of the general formula IV

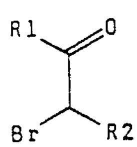

(IV)

in which R1 and R2 are as defined in Claim 1, and cyclisation of the compound of the general formula V thereby obtained

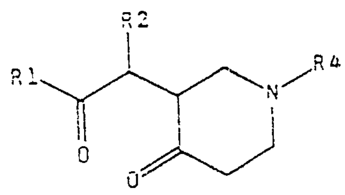

(V)

is performed so as to obtain a furopyridine of the general formula VI

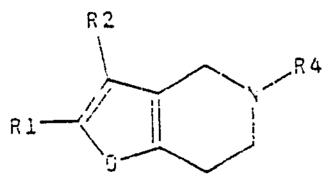

(VI)

and, when R4 denotes an acetyl or benzoyl group, the compound VI is subjected to the action of an acid or strong base to remove the acetyl group, or alternatively, when R4 denotes a benzyl group, and if so desired, the compound VI is subjected to catalytic hydrogenation.

3. Pharmaceutical composition, characterised in that it contains a compound according to Claim 1 in combination with a suitable pharmaceutical excipient.

**Claim** (for the Contracting State AT)

Process for preparing compounds corresponding to the general formula I

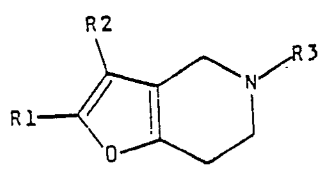

(I)

in which

R1 denotes a phenyl, halophenyl, methylphenyl, ethylphenyl, methoxyphenyl, trifluoromethylphenyl or naphthyl group,

R2 denotes hydrogen or a methyl or phenyl group, and

R3 denotes hydrogen or a benzyl group,

with the exception of the compounds in the formula of which R1 denotes a phenyl or 4-fluorophenyl group, R2 denotes hydrogen and R3 denotes hydrogen or a benzyl group, which process is characterised in that a 4-oxopiperidine of the general formula II

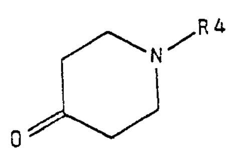

(II)

in which R4 denotes a benzyl, benzoyl or acetyl group, is subjected to the action of pyrrolidine, a compound of the general formula III

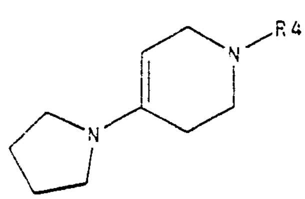

(III)

thereby being obtained, which is reacted with an α-brominated ketone of the general formula IV

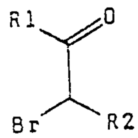

(IV)

in which R1 and R2 are as defined above, and cyclisation of the compound of the general formula V thereby obtained

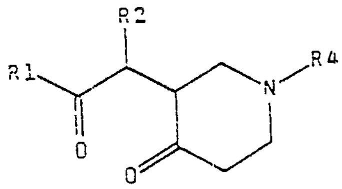

(V)

is performed so as to obtain a furopyridine of the general formula VI

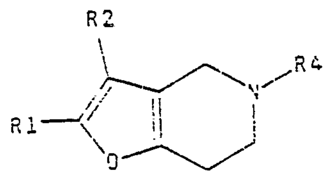

(VI)

and, when R4 denotes an acetyl or benzoyl group, the compound VI is subjected to the action of an acid or strong base to remove the acetyl group, or alternatively, when R4 denotes a benzyl group, and if so desired, the compound VI is subjected to catalytic hydrogenation.

**Patentansprüche** (für die Vertragsstaaten : BE, CH, DE, FR, GB, IT, LI, LU, NL, SE)

1. Verbindungen der allgemeinen Formel I

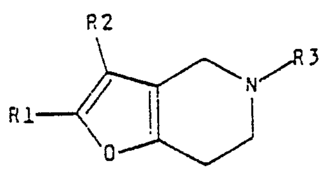

(I)

in welcher
R1 eine Phenyl-, Halogenphenyl-, Methylphenyl-, Ethylphenyl-, Methoxyphenyl-, Trifluormethylphenyl- oder Naphthylgruppe,
R2 Wasserstoff oder eine Methyl- oder Phenylgruppe und
R3 Wasserstoff oder eine Benzylgruppe bedeutet,
mit Ausnahme von Verbindungen, in deren Formel R1 eine Phenyl- oder 4-Fluorphenylgruppe, R2 Wasserstoff und R3 Wasserstoff oder eine Benzylgruppe darstellen, sowie deren Additionssalze mit pharmazeutisch verwendbaren Säuren.

2. Verfahren zur Herstellung von Verbindungen nach Anspruch 1, dadurch gekennzeichnet, daß man ein 4-Oxopiperidin der allgemeinen Formel II

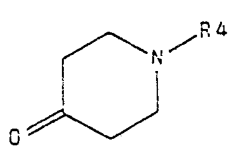

(II)

in welcher R4 eine Benzyl-, Benzoyl- oder Acetylgruppe darstellt, der Einwirkung von Pyrrolidin aussetzt, wodurch eine Verbindung der allgemeinen Formel III

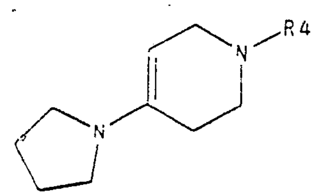

(III)

erhalten wird, die man mit einem α-bromierten Keton der allgemeinen Formel IV

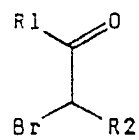

(IV)

in welcher R1 und R2 die in Anspruch 1 angegebene Bedeutung haben, reagieren läßt, worauf man die Zyklisierung der so erhaltenen Verbindung der allgemeinen Formel V

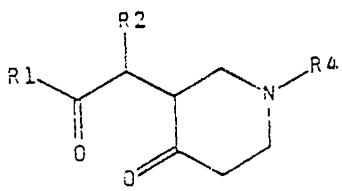

(V)

hervorruft, um ein Furopyridin der allgemeinen Formel VI

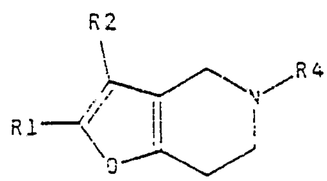

(VI)

zu gewinnen, und, wenn R4 für eine Acetyl- oder Benzoylgruppe steht, man die Verbindung VI der Einwirkung einer starken Säure oder Base aussetzt, um die Acetylgruppe zu eliminieren oder aber, wenn R4 eine Benzylgruppe darstellt und es gewünscht wird, die Verbindung VI einer katalytischen Hydrierung unterwirft.

3. Pharmazeutische Zusammensetzung, dadurch gekennzeichnet, daß sie eine Verbindung nach Anspruch 1 in Kombination mit einem geeigneten pharmazeutischen Träger enthält.

**Patentanspruch** (für den Vertragsstaat AT)

Verfahren zur Herstellung von Verbindungen der allgemeinen Formel I

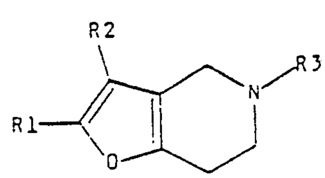

(I)

in welcher

R1 eine Phenyl-, Halogenphenyl-, Methylphenyl-, Ethylphenyl-, Methoxyphenyl-, Trifluormethylphenyl- oder Naphthylgruppe,

R2 Wasserstoff oder eine Methyl- oder Phenylgruppe und

R3 Wasserstoff oder eine Benzylgruppe bedeutet,

mit Ausnahme von Verbindungen, in deren Formel R1 eine Phenyl- oder 4-Fluorphenylgruppe, R2 Wasserstoff und R3 Wasserstoff oder eine Benzylgruppe darstellen, welches Verfahren dadurch gekennzeichnet ist, daß man ein 4-Oxopiperidin der allgemeinen Formel II

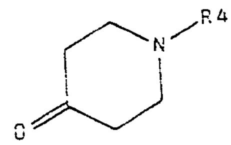

(II)

in welcher R4 eine Benzyl-, Benzoyl- oder Acetylgruppe darstellt, der Einwirkung von Pyrrolidin aussetzt, wodurch eine Verbindung der allgemeinen Formel III

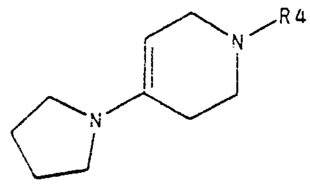

(III)

erhalten wird, die man mit einem α-bromierten Keton der allgemeinen Formel IV

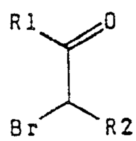

(IV)

in welcher R1 und R2 die oben angegebene Bedeutung haben, reagieren läßt, worauf man die Zyklisierung der so erhaltenen Verbindung der allgemeinen Formel V

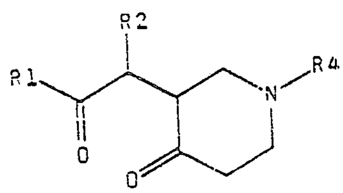

(V)

hervorruft, um ein Furopyridin der allgemeinen Formel VI

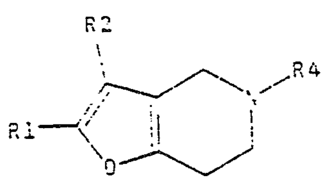

(VI)

zu gewinnen, und, wenn R4 für eine Acetyl- oder Benzoylgruppe steht, man die Verbindung VI der Einwirkung einer starken Säure oder Base aussetzt, um die Acetylgruppe zu eliminieren oder aber, wenn R4 eine Benzylgruppe darstellt und es gewünscht wird, die Verbindung VI einer katalytischen Hydrierung unterwirft.